# EUROPEAN PATENT SPECIFICATION

(11) **EP 2 919 649 B1**
(45) Date of publication and mention of the grant of the patent: **22.12.2021**
(21) Application number: 13792359.5
(22) Date of filing: 15.11.2013
(51) Int. Cl.: A61B 5/08, A61M 11/00, A61B 5/097

(54) **DEVICE AND METHOD FOR PULMONARY FUNCTION MEASUREMENT**
VORRICHTUNG UND VERFAHREN ZUR PULMONALEN FUNKTIONSMESSUNG
DISPOSITIF ET PROCÉDÉ DE MESURE DE LA FONCTION PULMONAIRE

(30) Priority: 15.11.2012 SE 1251298
(43) Date of publication of application: 23.09.2015
(73) Proprietor: JP Respiratory Technology AB, 232 51 Åkarp (SE)
(72) Inventor: LÖNDAHL, Jakob, 226 42 Lund (SE); WOLLMER, Per, 217 54 Malmö (SE)
(74) Representative: AWA Sweden AB
(86) International application number: PCT/EP2013/073977
(87) International publication number: WO 2014/076250

(56) References cited:
- WO-A2-02/09574
- US-A1- 2005 068 528
- None

## Description

### Technical Field of the Invention

This invention relates to a device for pulmonary function measurement in a subject to be used in the diagnosis of, e.g., emphysema or chronic obstructive pulmonary disease (COPD), where any method of diagnosis is not part of the claimed invention.

### Background

COPD is caused mainly by smoking but may also be induced by long-term exposure to chemical fumes, pollution or dust particles. COPD is characterised by persistent airflow limitation that is usually progressive and associated with an enhanced chronic inflammatory response to noxious particles or gases. The chronic airflow limitation is caused by a mixture of small airways disease (bronchiolitis) and parenchymal destruction (emphysema). There is also disease of the larger airways (bronchitis). COPD was ranked the sixth leading cause of death in 1990 and is projected to be the fourth leading cause of death worldwide by 2030 due to an increase in smoking rates and demographic changes in many countries. COPD is a progressive disease and there is no cure or method of reversal of the lungs' damaged state. The disease's progress may however be slowed down by better life style and medical treatment. After severe progression of COPD the common ultimate result and cause of death is heart failure due to the heart trying to cope with poor blood oxygenation (Calverley & Walker 2003). One of the dangerous features of COPD is that the symptoms are very weak at first and progress slowly as the disease evolves. At first, the shortness of breath can deceitfully be thought to originate from just poor physical fitness. Coughing can be thought to originate from a simple cold. Therefore, it is important to be able to diagnose patients suffering from COPD early.

Emphysema is an alveolar disease originating from inflammatory responses on alveolar surface, most often caused by smoke particles and deposition of tar while smoking tobacco, or by dust particles or chemical fumes. As excretion of inflammatory agents continues during longer periods, the thin walls between alveoli (the alveolar septa) are disintegrated. This changes the alveolar structure: the alveolar surface area is reduced, which reduces the transfer of gas between air and capillaries and, in addition, the removal of septa and their mechanical support relieves built-in alveolar elastic potential energy and expands the alveolar sacs. This creates even larger alveolar cavities. An immediate effect of this is that the lungs' elastic resting state upon expiration (functional residual capacity, FRC) is set to a larger volume and the feeling of a barrel chest is experienced. A healthy lung may expire air sufficiently through the lungs' elastic recoil by itself, but with emphysema the last air needs to be expired actively using abdominal and thoracic muscles. Gas exchange is severely impaired in advanced disease.

Chronic bronchitis and bronchiolitis is usually caused by inhalation of irritant particles, mainly from tobacco smoke, but also from air pollution, fumes and dust during long-term exposure. The irritants create inflammatory responses in the bronchi, making them swollen and inducing excretion of thick mucus layers. In addition, constrictions of smooth muscles surrounding the bronchi, bronchospasms, occur. All of these responses increase flow resistance in the airways making it more difficult to breathe. In order to remove all irritant particles and excessively excreted mucus, the body uses the action of coughing. As irritants are continuously supplied to the bronchi, this coughing tends to be chronic and hurtful for airways and involved muscles. Similar to emphysema, the bronchioles also lose their elasticity and become floppy and less capable of inflation (Braman 2006). Abnormalities in the peripheral airways (bronchioles and alveoli) occur at an early stage of COPD and are therefore important to identify for an early diagnosis to be made.

Diagnosis of emphysema and bronchitis can be made through regular lung function measurements. Spirometry is one of the most basic technical ways of diagnosing pulmonary disorders. The easiest setup requires only a flow meter, through which a subject breathes, and which data is logged from. Spirometry measures the volume of a maximal breath (vital capacity, VC) and the forced expiratory volume during the first second of expiration (FEV₁). Spirometry detects airflow limitation, but does not provide any specific information about abnormalities in peripheral airways. Total lung capacity (TLC), FRC and residual volume (RV) may be measured by e.g. body plethysmography. In emphysema, TLC may be increased, but this is seen late in the development of the disease. RV and FRC are increased in COPD, but these measurements do not distinguish between disease in central or peripheral airways.

Emphysema and bronchitis may also be diagnosed through more expensive scanning methods, such as computed tomography (CT) or magnetic resonance imaging (MRI) with hyperpolarized gas (e.g. Swift et al 2005).

An aerosol is a colloid suspension of fine solid particles or liquid droplets in a gas, for example clouds and smoke. The four main means by which aerosols leave their carrier gas to eventually deposit onto their surrounding surfaces are: impaction, interception, sedimentation and diffusion (Ferron 1994). The importance of each of these means is in turn dependent on factors such as particle size, gas velocity, temperature, and channel dimensions. Aerosol impaction refers to the instance of a particle leaving its carrier gas and impacting onto the surrounding channel surface in a sharp bend of the carrier gas flow. Several models on deposition by impaction in human airways have been made, and they postulate that impaction is mainly affecting particles larger than 1 µm (Agnew et al 1984). These large particles then deposit almost exclusively in the nasopharyngeal and bronchial region where air velocities are high and directional changes are abrupt. Aerosol interception refers to deposition of aerosols upon entry to narrow channel pathways. Interception deposition is entirely due to bulky physical interaction with channel walls and is primarily of importance for fibre like particles. Aerosol sedimentation is deposition due to gravitational forces. Probability of sedimentation is larger for larger particles and in horizontally oriented airways with small diameters. Sedimentation is more effective in smaller airways and in the alveolar region. For aerosol particles with diameter below 0.5 µm, sedimentation is of small or no importance. Aerosol deposition by diffusion refers to particles deviating from their carrier gas and hitting surrounding channel walls by Brownian motion. Diffusive deposition in human airways is confined to the peripheral airways (bronchioles and alveoli). This type of deposition is low or almost zero for particles with diameters of more than about 0.5 µm (Hinds 1999), but very prominent and exclusive for nano-sized particles compared to other mechanisms of deposition.

Differences of particle deposition between diseased and healthy lungs mainly result from gradual changes in lung morphology and continuous adaptations of lung volumes and breathing patterns to the modified lung architecture. In emphysema, the abnormally enlarged alveolar sizes influence particle behaviour in the alveoli to an appreciable extent.

In the research article Peripheral Airspace Dimensions in patients with COPD (Beinert et al 1995) a type of measurement called ADAM (aerosol derived airway morphometry), a method which could be used to evaluate enlarged airspace dimensions in patients with COPD, is described. In an ADAM measurement, the lungs are filled with a monodisperse aerosol. The patient holds his or her breath for a certain time and the particles settle onto the airways. The reduction of aerosol concentration in a certain volume element is measured in the expired air. The ratio of particle concentration in the exhaled volume elements versus the concentration in the inhaled aerosol decreases with increasing breath holding time. This is used in turn to measure effective airspace dimension. The particles used in this study were 890 nm in diameter. To reduce the dead space in the mouth cavity of the patient, a dental compound covered in silicone filling up a part of the mouth is used during the measurements. This may be uncomfortable to the patient. Furthermore, in ADAM-measurements, it is required that aerosol is inhaled several times and to different depths of the airways. This is very cumbersome and complicated and requires substantial participation of the patient. The deposition of particles close to 1 µm is not only influenced by sedimentation, but also by impaction. If upper airways are distorted or if breathing flows are altered due to a lung disease, the deposition is thus likely to be influenced. The deposition of these particles is thus not only a measure of alveolar volumes. For example, in individuals with severe disease of the bronchi, there will be losses due to impaction and thus no clear or reliable information on the status of the peripheral airways is obtained.

WO 02/09574 A2 discloses controlled delivery of medicament to the respiratory system of a patient, a measured quantity of a medicament, i.e., a bolus is introduced into the inspiratory flow stream of a patient, via a breathing tube, and inhaled by the action of the patient's breathing cycle. In the course of a breathing cycle of the patient, numerous properties or characteristics of the medicament, i.e., the bolus, the inspiratory flow or characteristics of the expiratory flow, and/or other useful information are derived. The derived information is employed to control subsequent delivery of boli to the patient, including the delivery of each bolus as a function of the commencement or progression of the breathing cycle, as input for calculations or determinations which are useful in analyzing the effectiveness of delivery of the medicament to the patient, patient compliance, direction of flow through the breathing tube, and other uses.

An object of the present invention is to overcome these problems.

### Summary of the Invention

According to a first aspect of the invention, the above and other objects of the invention are achieved, in full or at least in part, by a device as defined by claim 1. Further embodiments of the inventive device are disclosed in the depended claims 2-10. According to claim 1 the above object may be achieved by a device for pulmonary function measurement in a subject comprising a means for providing an aerosol comprising particles to be inhaled by the subject and a means for analysis of a portion of air exhaled by the subject. In this way, the characteristics of the exhaled particles can be carefully monitored. The particles are hydrophobic and thus, the particles do not absorb water and increase in size to a large extent in the upper airways, but are instead transported with the inhaled air to the peripheral airways. The particles are within the range of 20 - 200 nm, preferably 50 - 100 nm. Particles of this size are able to enter even the smaller structures of the airways and can provide detailed information of the status of the peripheral airways of the subject. The pulmonary function measurement may also provide information on dimensions of the peripheral airways. The portion which is analysed is exhaled after exhalation of a volume corresponding to total dead space. The total dead space corresponds to the volume from the airway opening to the gas exchanging region of the lungs plus the volume of air in the device which is inhaled with the ensuing breath. Thus, in this context, the total dead space corresponds to the volume of the respiratory system that is ventilated but which has no gas exchange and the volume of air in the device which is inhaled with the ensuing breath. The aerosol exhaled from the dead space has thus not entered the peripheral airways. In this way, only the particles which have entered the peripheral airways are analysed.

The means for providing an aerosol comprising particles to be inhaled may comprise a reservoir providing a uniform concentration/distribution of particles in the aerosol, which gives the possibility to monitor and control the amount of particles which is to be inhaled by the subject.

According to an embodiment, the means for providing an aerosol comprising particles to be inhaled also comprises a reservoir having an aperture enabling gas to flow into the reservoir upon inhalation. The inflow of gas replaces the aerosol inhaled and maintains the breathing resistance at an essentially constant level. This also ensures that the particles in the aerosol remain uniformly distributed, and do not agglomerate or adhere to the walls of the reservoir.

According to another embodiment, the device may further comprises a collector for collecting the exhaled air. The collector comprises means for heating exhaled air in order to avoid condensation from the subject's breath, since such condensation could possibly interfere with the measurement of the amount of exhaled particles.

According to another embodiment, the collector is arranged to maintain the exhaled air essentially in a laminar flow regime, since a turbulent flow regime would risk mixing the exhaled air, making the measurement of the correct portion of exhaled air impossible.

According to yet another embodiment, the device further comprises means for providing air for inhalation, which is free of particles with the same characteristics as the particles comprised in the aerosol to be inhaled. This has the advantage that particles from the surrounding air, with the same characteristics as the particles in the aerosol to be inhaled, do not interfere with the measurements.

The device further comprises means for determining the amount of particles in the aerosol to be inhaled, and the means for analysis of exhaled air comprises means for determining the amount of particles in the exhaled air. By comparing the amount of particles in the aerosol to be inhaled to the amount of particles in the exhaled air, the amount of particles that has been deposited in the airways of the subject can be determined. This amount will be an indirect measure of the status of the airways of the subject. In emphysema, the abnormally enlarged alveolar sizes will lead to a decreased amount of deposited particles of some particle sizes. During normal breathing inhaled particles in the approximate range 0.01-10 µm have a significant (typically > 10%) probability to reach the alveolar region, although above 1 µm a larger fraction is deposited in the head airways. If there is no airflow, particles that are larger than around 0.5 µm deposit mainly by gravitational settling. These particles have a decreased probability to deposit in enlarged alveoli due to increased settling distances. Particles smaller than around 0.5 µm mainly collide with nearby surfaces and deposit due to diffusion by Brownian motion. These particles have a decreased probability to deposit in enlarged alveoli due to increased diffusion distances.

The means for providing the aerosol comprising particles to be inhaled may be arranged to provide a concentration of particles of less than 500,000 particles/cm³, preferably 1,000 to 50,000 particles/cm³. A high concentration of particles is needed in order to give a low error of measurement. However, if the concentration is too high, the measured changes in particle concentration will not only be due to deposition but also coagulation. In addition, particle coagulation will alter particle size and thereby change deposition probabilities.

According to one embodiment, the device further comprises means for drying the aerosol to below 20 % relative humidity. This reduces condensation of water in the tubing and also reduces a change in sample volume due to water vapour.

According to the invention, the device further comprises a system of valves being openable to the subject, being openable to the means for providing air for inhalation, being openable to the reservoir, and being openable to the means for determining the amount of particles in inhaled aerosol and exhaled air. Furthermore, the system of valves is adjustable to either allow passage of air from the means for providing air for inhalation to the subject, allow passage of aerosol comprising particles from the reservoir to the subject, allow passage of aerosol comprising particles from the reservoir to the means for determining the amount of particles in the aerosol, allow passage of exhaled air from the subject to the means for determining the amount of particles in exhaled air, or allow passage of exhaled air from the subject to the surroundings. This system of valves makes it possible to control the kind of air the subject inhales, air which is free of particles with the same characteristics as the particles comprised in the aerosol to be inhaled, or the aerosol comprising particles. In addition, this system of valves makes it possible to control which portion of the exhaled air is directed to the means for determining the amount of particles in exhaled air.

The system of valves may be adjustable to prevent inhaled aerosol from being exhaled by the patient, i.e. controlling the period of time the subject holds his or her breath, or ensuring that no air is allowed to pass to the means for determining the amount of particles in exhaled air before its predetermined residence time in the airways of the subject has passed.

Not according to the invention, the device is used for diagnosis and/or monitoring of pulmonary disease in a subject. Compared to the methods used today for the diagnosis and/or monitoring of pulmonary disease, the device will not require any supplemental measurements to provide significant results.

According to a yet another aspect not covered by the invention, a method for pulmonary function measurement in a subject is also provided, wherein the subject inhales and exhales through a mouthpiece. The method comprises the steps of providing aerosol comprising particles to be inhaled by the subject, wherein the particles are within a range of 20-200 nm, preferably 50-100 nm, and analysing air exhaled by the subject. An advantage of this method is that the particles are small enough to reach the peripheral airways and analysis of the deposition of these particles in the airways will provide information about the status of these regions of the airways of the subject. Furthermore, the method may comprise the step of the subject inhaling and exhaling according to a predefined pattern. This secures that each measurement is performed in essentially the same manner, making it possible to compare the results from different measurements or even from different subjects. In addition, the method further comprises the step of the subject holding his/her breath for a period of time, ensuring that the particles reach the peripheral airways. In another variant, the method further comprises the step of the subject holding his/her breath for 5 to 10 seconds. In a preferred variant, the predefined pattern resembles the breathing pattern used in other methods measuring lung function. Thus, subjects suffering from e.g. COPD are already used to it, minimizing the risk of having to repeat the measurements due to the wrong execution of the procedure.

The method may further comprise the steps of generating an aerosol comprising particles smaller than 200 nm; determining an amount of the particles in the aerosol; directing air through the mouthpiece to the subject for a period of time, wherein the air is free of particles of the same size as the particles comprised in the aerosol to be inhaled; the subject exhaling completely; directing aerosol comprising particles through the mouthpiece to the subject; the subject inhaling; the subject holding his/her breath for a period of time; the subject exhaling; collecting a specific volume of the air exhaled by the subject, preferably from the alveolar region after exhalation of a volume corresponding to dead space; determining an amount of the particles in the air exhaled by the subject; determining a difference between the particle amount in the exhaled air and the particle amount in the inhaled aerosol; and comparing the difference with reference values. This method ensures that the measurements are conducted in the same manner every time, giving reliable and comparable results. Furthermore, the method may also comprise the step of heating the exhaled air, to avoid the condensation of water vapour on the exhaled aerosol particles and subsequent interference of the measurement of the amount of particles in the exhaled air. Additionally, condensation could damage the instrument itself.

According to another aspect of the present disclosure, a control system for a device for pulmonary function measurement in a subject is also provided.

Furthermore, the method may comprise the step of the subject inhaling and exhaling according to a predefined pattern. This secures that each measurement is performed in essentially the same manner, making it possible to compare the results from different measurements or even from different subjects. In addition, the method further comprises the step of the subject holding his/her breath for a period of time, ensuring that the particles reach the peripheral airways. In another variant, the method further comprises the step of the subject holding his/her breath for 5 to 10 seconds. In a preferred variant, the predefined pattern resembles the breathing pattern used in other methods measuring lung function. Thus, subjects suffering from e.g. COPD are already used to it, minimizing the risk of having to repeat the measurements due to the wrong execution of the procedure.

The method may further comprise the steps of generating an aerosol comprising particles smaller than 200 nm; determining an amount of the particles in the aerosol; directing air through the mouthpiece to the subject for a period of time, wherein the air is free of particles of the same size as the particles comprised in the aerosol to be inhaled; the subject exhaling completely; directing aerosol comprising particles through the mouthpiece to the subject; the subject inhaling; the subject holding his/her breath for a period of time; the subject exhaling; collecting a specific volume of the air exhaled by the subject, preferably from the alveolar region after exhalation of a volume corresponding to dead space; determining an amount of the particles in the air exhaled by the subject; determining a difference between the particle amount in the exhaled air and the particle amount in the inhaled aerosol; and comparing the difference with reference values. This method ensures that the measurements are conducted in the same manner every time, giving reliable and comparable results. Furthermore, the method may also comprise the step of heating the exhaled air, to avoid the condensation of water vapour on the exhaled aerosol particles and subsequent interference of the measurement of the amount of particles in the exhaled air. Additionally, condensation could damage the instrument itself.

According yet another aspect not covered by the invention, a control system for a device for pulmonary function measurement in a subject is also provided.

Preferably, the control system comprises means for identifying when the subject has exhaled completely; means for setting the system of valves so that the subject inhales from the aerosol reservoir; means for identifying when the subject has stopped inhaling; means for shutting all openings in the system of valves; means for opening the system of valves to the means for determining the amount of particles in inhaled aerosol and exhaled air; means for measuring the volume of exhaled air; means for shutting the system of valves to the means for determining the amount of particles in inhaled aerosol and exhaled air after a volume has been exhaled; means for measuring an amount of particles in the exhaled air; means for comparing a difference between the amount of particles in exhaled aerosol and inhaled air; and means for calculating a ratio of said difference to a reference value. This control system enables the control of what is inhaled by the subject, air which is free of particles with the same characteristics as the particles comprised in the aerosol to be inhaled, or the aerosol comprising particles. In addition, this control system makes it possible to hinder air to pass from the subject to the means for determining the amount of particles in exhaled air, to control which portion of the exhaled air is directed to the means for determining the amount of particles in exhaled air, and finally provide a measurement corresponding to the amount of deposited particles in the airways of the subject. The control system also makes it possible to control the duration of breath-hold.

Other objectives, features and advantages of the present inventive concept will appear from the following detailed disclosure, from the attached claims, as well as from the drawings.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

### Brief Description of the Drawings

By way of example, embodiments of the present inventive concept will now be described with reference to the accompanying drawings, in which:
Figure 1 is a schematic view of one embodiment,
Figure 2 is a schematic drawing of one breathing pattern of the subject during one measurement,
Figure 3 is a schematic drawing of a system of valves according to an embodiment, and
Figure 4 shows the fundamental steps performed by the device.
Figure 5 is a schematic illustration of the system of valves and airflows during the measurement cycle.

### Detailed Description of Preferred Embodiments

The invention relates to a device for pulmonary function measurement in a subject. The subject may be an animal or a human. The subject may be healthy or suspected to suffer from, or diagnosed with, a pulmonary disease or disorder such as a smoking-induced lung disease, COPD or emphysema, bronchiectasis, asthma or interstitial lung disease. The subject may also be an athlete.

In short, the subject inhales an aerosol comprising particles, holds his/her breathe for a period of time and exhales. It is to be understood that the exhaled air may comprise aerosol. The amount of particles in the inhaled aerosol and exhaled air are measured and compared. The difference between the concentrations in inhaled aerosol and exhaled air reflects the structure of the alveoli and may be used as a means to diagnose pulmonary disease. Instead of, or in addition to, air another gas may be used, e.g. air, a specific gas, or a combination of one or more different specific gases with or without air. A specific gas may e.g. be carbon monoxide (CO), helium (He), nitric oxide (NO) or methane (CH₄). When using CO, the diffusion of CO from the inhaled gas to the blood may be analysed simultaneously with the analysis of the amount of particles in the inhaled aerosol and exhaled air. Uptake of CO or NO into the blood depends on diffusion within the airspace and across the alveolar-capillary barrier. The combination of measurements with nanoparticles and CO or NO may therefore provide important information concerning gas transfer across the alveolar-capillary barrier.

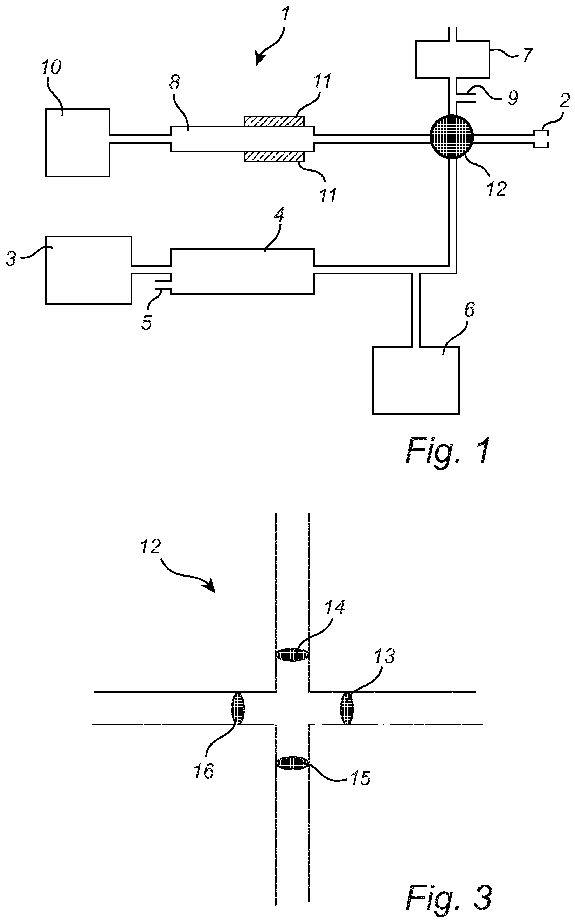

One embodiment will now be described in relation to Figures 1 to 4.

The device 1 comprises a mouthpiece 2 for inhaling and exhaling. The mouthpiece 2 can be made of any material suitable for human use, such as plastic, rubber, metal or another suitable material. Preferably it should be made of a conducting material to avoid excessive particle losses from electrostatic forces. The mouthpiece 2 may be expendable or reusable.

The device 1 comprises a means 3 for the generation of aerosols. In one embodiment, the aerosol is generated using an electrospray, but other means may also be used, such as a nebulizer/atomizer, or a device based on spark discharge. The aerosol may also be generated by mechanical interaction, bubble bursting, combustion or evaporation and nucleation. In one embodiment, an aerosol with stable particle concentration over time is generated continuously with techniques based on, for instance, atomization/nebulization, electrospray or nucleation. Stability of the aerosol concentration may be improved by controlled time-dependent dilution. Alternatively, the aerosol is generated into a mixing chamber. The mixing chamber may be both flexible (e.g. a bag) or rigid (e.g. stainless steel) with a continuous flow of aerolsol.

Optionally, if the generated aerosol is not monodisperse, a specific size fraction may be selected before the particles are allowed to enter the reservoir 4. This can be achieved by using, e.g., a differential mobility analyzer (DMA) which allows only the particles of the right size to pass. Alternatively, only the size fraction(s) of interest may be measured from an inhaled polydisperse aerosol. The particles have a uniform concentration/distribution in the aerosol, which is achieved in a well-mixed reservoir 4. This may be accomplished e.g. by a constant flow of aerosol through the reservoir or by a mixing fan.

The aerosols are passed into the reservoir 4. In one embodiment, the reservoir 4 is a combination of a flexible anti-static bag and a stainless steel reservoir tank. To achieve a stable aerosol suitable for inhalation the reservoir 4 may be flexible, and thereby adaptable to atmospheric pressure. It may also be built to allow a continuous flow of aerosol. The continuous flow of aerosol prevents concentration changes that will otherwise occur by deposition and coagulation if the aerosol is allowed to reside in the reservoir 4 for some time. The maximum volume of the reservoir 4 is preferably between 7 and 15 L, typically 10 L. Upon aerosol inhalation, aerosol is removed from the reservoir 4 and its flexible volume is decreased.

The size of the particles in the aerosols is typically between 20 and 200 nm, preferably 50 -100 nm, and the concentration of particles in the aerosol to be inhaled is less than 500,000 particles/cm³, preferably 1,000 - 50,000 particles/cm³. The desired concentration of particles may be achieved by e.g. diluting the aerosol with particle-free gas, by use of a particle filter or by use of an electric field to decrease particle concentration. If the concentration is too high, the particles will aggregate and will not be suitable for the measurement of pulmonary function. However, if the concentration of particles is too low, the error of measurement will be high.

The particles may be of any material suitable for use in a human or animal subject, e.g polystyrene latex, Di-Ethyl-Hexyl-Sebacat (DEHS), stearic acid or other hydrophobic material with low toxicity. The particles may comprise radioactive, luminescent, fluorescent or phosphorescent components, such as ^{99m}Tc or any other radioactive isotope suitable for human use, fluorescein or any other fluorescent dye suitable for the intended use.

The concentration in the reservoir 4 is measured by means 6 for determining the content or amount of particles in aerosol to be inhaled. In one embodiment, the amount is measured using a condensation particle counter (CPC), but may also be determined by other instruments such as an electrometer. For instance, an electrostatic precipitator may be used. Other means of determining the amount of particles in the aerosol may be by measuring the particles' radioactivity, luminescence, fluorescence, phosphorescence or the like. The sampling point for the measurement should be as close to the mouthpiece as possible to minimize correction for particle losses in the tubing. The aerosol should preferably be dried to below 20% relative humidity in order to reduce condensation of water in the tubing and also to reduce a change in sample volume due to water vapour. The aerosol may be dried before entering the means 6 for determining the content or amount of particles in aerosol.

The device 1 also comprises means 7 for providing air to the subject. Preferably, the air provided to the subject is free of particles having the characteristics of the particles comprised in the aerosol. In one embodiment, the particle-free air from a pump is used, but other means for providing particle-free air, such as filtered room air, may also be used.

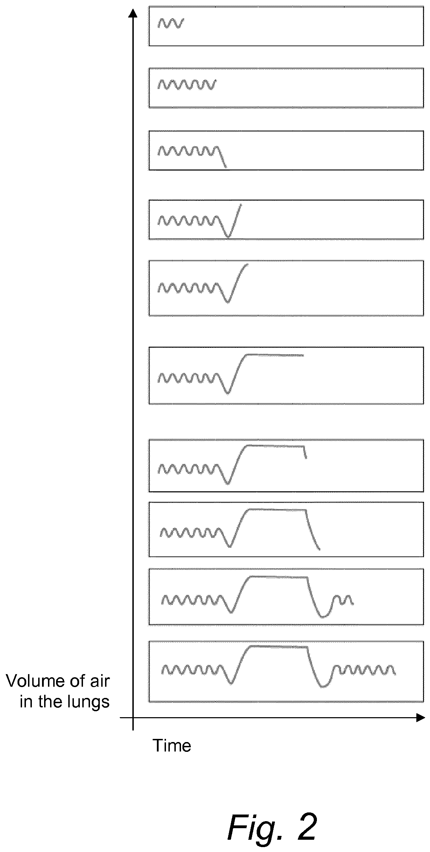

During a measurement, the subject follows a predefined breathing scheme and in Fig. 2, one example of such a breathing pattern during one measurement is shown. The scheme starts with the subject breathing air, which is devoid of particles with the characteristics of the particles comprised in the aerosol, normally. After a couple of breaths the test can start. The subject exhales completely and after this, the subject inhales from the aerosol reservoir 4 and the lungs are filled. The subject holds his/her breath for a period of time. In a preferred embodiment this time interval is 10 seconds. Thereafter, the subject exhales.

A volume of the exhaled air is collected in a collector 8. The collector 8 is arranged to minimize mixing of the exhaled aerosol. Importantly, the mixing of aerosol in the collector 8 should be low, since the air sampled from a specific part of the collector 8 should contain air from a specific part of the exhaled air. The particles are sampled at a volumetric lung depth, i.e. from a part of the aerosol after exhalation of a specific volume, typically between 0.5 and 4 L, preferably 1,5 L. Alternatively, the desired fraction of exhaled aerosol could be directed into a sampling chamber by use of valves in the exhalation part of the breathing circuit. Further, the particle concentration may be monitored continuously in the exhaled air. The rest of the exhalation may be performed through the port 14 open to a clean air supply 9.

The amount of particles in the exhaled air is determined by a means 10 for analysis of air exhaled from the subject. Preferably, the aerosol is dried before determining the amount of the particles in the exhaled air in order to reduce condensation of water in the tubing and also to reduce a change in sample volume due to water vapor. The aerosol may be dried before entering the means 10 for analysis of air exhaled from the subject. In one embodiment, the amount is measured using a condensation particle counter (CPC), but may also be determined by other instruments based on light scattering. Alternatively, an electrostatic precipitator may be used. Other means of determining the amount of particles in the aerosol may be by measuring the particles' radioactivity, luminescence, fluorescence, phosphorescence or the like. If two individual detectors (6, 10) are used to measure the concentration in the reservoir 4 and to determine the amount of particles in the exhaled air, careful calibration of the two detectors is required to verify that they have similar sensitivity. Preferably, the same instrument is used as the means 6 for determining the amount of particles in aerosol to be inhaled and the means 10 for analysis of air exhaled from the subject. A valve is then added to the detector to switch between the reservoir 4 and the collector 8 for exhaled air.

The exhaled air is preferably heated before it enters the means 10 for analysis of exhaled air in order to avoid condensation of the vapour in the exhaled air. The exhaled air is heated by means 11 for heating exhaled air. In one embodiment, the exhaled air is heated to a temperature above 34 °C. However, when determining the temperature, the possible subsequent heating of the mouthpiece 2 must be taken into account to assure that the subject is not subjected to any unnecessary discomfort during the measurement. Due to this, the temperature limit is partly set by the materials of the different components of the device, especially the mouthpiece 2. The aerosol should preferably be dried to below 20% relative humidity before measurement in order to reduce condensation of water in the tubing and to reduce a change in sample volume due to water vapour.

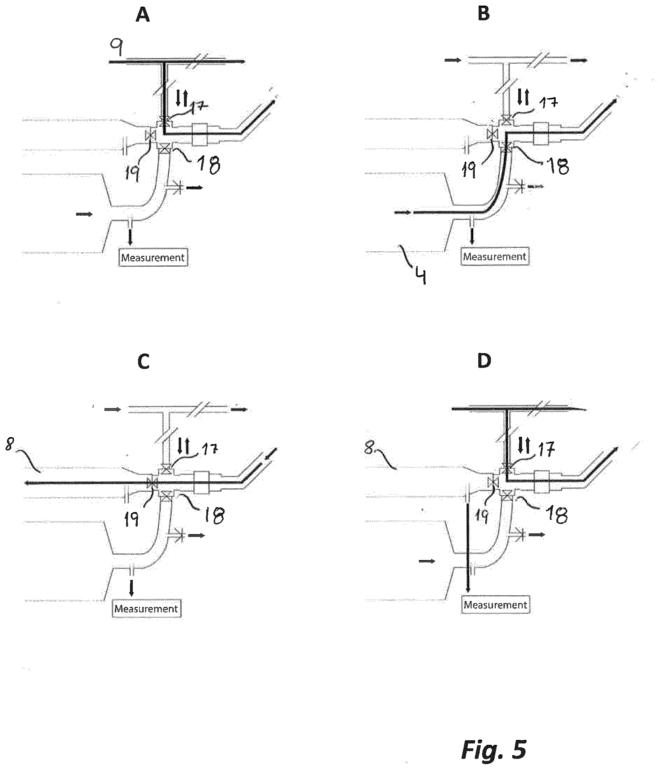

The airflows are controlled by a system of valves 12 shown in Fig. 3. It is advantageous if each of the valves in the system of valves 12 has a short reaction time. In a preferred embodiment the system of valves 12 is a 4-port balloon valve, but other types of valves may also be used, such as solenoid valves. A first port 13 opens to the subject. A second port 14, with a valve 17 (see Fig. 5), opens to the means 7 for generation of air for inhalation, a third port 15 with a valve 18 (see Fig. 5) opens to the reservoir 4, and a fourth port 16 with a valve 19 (see Fig. 5) opens to the means 6 for determining the concentration of particles in inhaled aerosol and in exhaled air. The valves are adjustable to either allow passage of air from the means 7 for generation of air for inhalation to the subject, allow passage of aerosol comprising particles from the reservoir 4 to the means 6 for determining the amount of particles in aerosol to be inhaled, allow passage of aerosol comprising particles from the reservoir 4 to the subject, or allow passage of exhaled air from the subject to the means 10 for determining the amount of particles in exhaled air and allow passage of exhaled air from the subject to the surroundings.

Breathing flow may be monitored with a flow meter placed either at the mouthpiece 2 or several flow meters placed at the reservoir 4 and the collector 8 and at the port 14 open to the clean air supply 9. A flow meter at the mouthpiece 2 has the advantage of a less complex set-up. Flow meters at the reservoir 4 and the collector 8 have the advantage that the particle losses between the sampling points for inhaled aerosol and exhaled air can be reduced.

The tubing is preferably metallic or of any other conducting material, in order to reduce adherence caused by static electricity.

In addition to being used for pulmonary function measurements, a device may be used to evaluate the behaviour of different kinds of particles, toxic and non-toxic in the airways. Furthermore, a device may be used to measure and validate the function of different kinds of devices for measurement of pulmonary function. In such case, the human or animal subject will be replaced by a pump. A device of the type here described may also be used for determining respiratory tract deposition of air pollutions and the like.

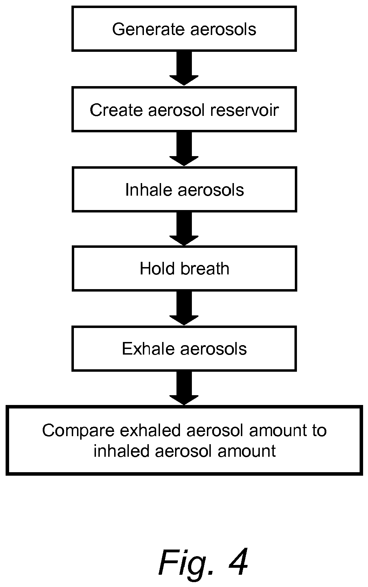

In Fig. 4, the fundamental steps performed by the device are shown.

The device for pulmonary function measurement described above is controlled by a control system comprising means (e.g. a flow meter) for identifying when the subject has exhaled completely or for monitoring of the subject's breathing; means (e.g. a computer program) for setting the system of valves 12 to direct inhalation of aerosol from reservoir 4; means (e.g. a flow meter) for identifying when the subject has stopped inhaling; means (e.g. computer-controlled electronics) for shutting all openings in the system of valves 12; means (e.g. computer-controlled electronics) for opening the system of valves 12 to the means 6 and 10 for determining the amount of particles in inhaled aerosol and exhaled aerosol; means (e.g. flow meters) for measuring the volume of exhaled air; means (e.g. computer-controlled electronics) for shutting the system of valves 12 to the means 6 and 10 for determining the amount of particles in inhaled aerosol and exhaled air after a volume has been exhaled; means 10 for measuring an amount of particles in the exhaled air; means (e.g. a computer program) for comparing a difference between the amount of particles in exhaled aerosol and inhaled air; and means (e.g. a computer program) for calculating a ratio of said difference to a reference value.

Fig. 5 is a schematic illustration of the system of valves 12 and airflows during the measurement cycle. During the initial phase of the measurement cycle (see Fig. 2) air is drawn from the inlet for particle free air (the clean air supply 9). In the system of valves 12, the valve 17 connected to particle free air is open while the other two valves 18 and 19 are closed (Fig. 5A). In the next step, after a maximum exhalation, particles are inhaled. In the system of valves 12, the valve 18 connected to the container for inhalation of aerosol (the reservoir 4) is open while the other two valves 17 and 19 are closed (Fig. 5B). After the inhalation of aerosol particles all three valves 17, 18 and 19 are closed for a desired amount of time, typically 10 seconds. Thereafter the valve 19 connected to the container for exhaled aerosol (the collector 8 for collecting exhaled air) is opened while the other two valves 17 and 18 are closed (Fig. 5C). After exhalation of a desired volume of aerosol, typically around 1.5 L, the valve 19 connected to the container for exhaled aerosol is closed. At the same time the valve 17 to the particle free air is opened to allow continued exhalation and breathing. Aerosol is sampled from the container for exhaled aerosol (Fig. 5D).

It will be appreciated that the inventive concept has been illustrated with reference to exemplary embodiments, and that the invention can be varied in many different ways within the scope of the claims.

It should also be noted that the inclusion in the appended claims of some of the numerals used in the figures is purely for illustrative purposes and not to be considered as limiting the scope of the claims.

### References

AGNEW, J. E., PAVIA, D. & CLARKE, S. W. 1984. Aerosol particle impaction in the conducting airways. Phys Med Biol, 29, 767-77.

BEINERT, T., BRAND, P., BEHR, J., VOGELMEIER, C. & HEYDER, J. 1995. Peripheral airspace dimensions in patients with COPD. Chest, 108, 998-1003.

BRAMAN, S. S. 2006. Chronic cough due to chronic bronchitis: ACCP evidence-based clinical practice guidelines. Chest, 129, 104S-115S.

CALVERLEY, P. M. & WALKER, P. 2003. Chronic obstructive pulmonary disease. Lancet, 362, 1053-61.

FERRON, G. A. 1994. Aerosol properties and lung deposition. European Respiratory Journal, 7, 1392-4.

HINDS, W. C. 1999. Aerosol technology: properties, behavior, and measurement of airborne particles, New York, Wiley.

SWIFT, A. J., WILD, J. M., FICHELE, S., WOODHOUSE, N., FLEMING, S., WATERHOUSE, J., LAWSON, R. A., PALEY, M. N. & VAN BEEK, E. J. 2005. Emphysematous changes and normal variation in smokers and COPD patients using diffusion 3He MRI. Eur J Radiol, 54, 352-8.

## Claims

1. A device (1) for pulmonary function measurement in a subject, the device comprising
means (3) for generation of aerosols comprising hydrophobic particles within the range of 20 - 200 nm, preferably 50 - 100 nm, to be inhaled by the subject, the means (3) for generating an aerosol comprises a reservoir (4) providing a uniform concentration/distribution of particles in the aerosol, wherein the means (3) is configured to pass the generated aerosols to the reservoir (4),
means (6, 10) for determining the amount of particles in inhaled aerosol and exhaled air, and
a system of valves being openable to the subject, being openable to a means (7) for providing air for inhalation, being openable to the reservoir (4), and being openable to the means (6, 10) for determining the amount of particles in inhaled aerosol and exhaled air,
wherein the system of valves makes it possible to control which portion of exhaled air is directed to the means (10) for determining the amount of particles in exhaled air, wherein said portion is exhaled after exhalation of a volume corresponding to dead space.

2. A device (1) according to claim 1, the reservoir (4) having an aperture (5) enabling gas to flow into the reservoir (4) upon inhalation.

3. A device (1) according to claim 1 or 2, further comprising a collector (8) for collecting the exhaled air, said collector (8) comprising means (11) for heating exhaled air.

4. A device (1) according to any one of the preceding claims, further comprising a collector (8) for collecting the exhaled air.

5. A device (1) according to any one of the preceding claims, wherein said air for inhalation provided by said means (7) for providing air for inhalation is free of particles with the same characteristics as the particles comprised in the aerosol to be inhaled.

6. A device (1) according to any one of the preceding claims, the means (6, 10) for determining the amount of particles in the inhaled aerosol and the exhaled air is the same instrument.

7. A device (1) according to any one of the preceding claims, wherein said means (3) for providing the aerosol comprising particles to be inhaled is arranged to provide a concentration of particles of less than 500,000 particles/cm³, preferably 1,000 to 50,000 particles/cm³.

8. A device (1) according to any one of the preceding claims, further comprising means for drying the aerosol to below 20% relative humidity.

9. A device (1) according to any one of the preceding claims, wherein the system of valves (12) is adjustable to either allow passage of air from the means (7) for providing air for inhalation to the subject, allow passage of aerosol comprising particles from the reservoir (4) to the subject, allow passage of aerosol comprising particles from the reservoir (4) to the means (6) for determining the amount of particles in the aerosol, allow passage of exhaled air from the subject to the means (10) for determining the amount of particles in exhaled air, or allow passage of exhaled air from the subject to the surroundings.

10. A device (1) according to claim 9, wherein the system of valves (12) is adjustable to prevent inhaled aerosol to be exhaled by the subject.

## Patentansprüche

1. Vorrichtung (1) zur Lungenfunktionsmessung in einem Subjekt, wobei die Vorrichtung umfasst:
Mittel (3) zur Erzeugung von Aerosolen, die hydrophobe Partikel in dem Bereich von 20-200 nm, vorzugsweise 50-100 nm, umfassen, zur Inhalation durch das Subjekt, wobei das Mittel (3) zur Erzeugung eines Aerosols ein Reservoir (4) umfasst, das eine gleichmäßige Konzentration/Verteilung von Partikeln in dem Aerosol bereitstellt, wobei das Mittel (3) dafür gestaltet ist, die erzeugten Aerosole zu dem Reservoir (4) weiterzugeben,
Mittel (6, 10) zur Bestimmung der Menge von Partikeln in inhaliertem Aerosol und ausgeatmeter Luft und
ein System von Ventilen, das zu dem Subjekt geöffnet werden kann, zu einem Mittel (7) zur Bereitstellung von Luft zur Inhalation geöffnet werden kann, zu dem Reservoir (4) geöffnet werden kann und zu den Mitteln (6, 10) zur Bestimmung der Menge von Partikeln in inhaliertem Aerosol und ausgeatmeter Luft geöffnet werden kann,
wobei es das System von Ventilen ermöglicht, zu steuern, welcher Teil von ausgeatmeter Luft zu dem Mittel (10) zur Bestimmung der Menge von Partikeln in ausgeatmeter Luft geleitet wird, wobei der Teil nach Ausatmen eines Volumens, das Totraum entspricht, ausgeatmet wird.

2. Vorrichtung (1) gemäß Anspruch 1, wobei das Reservoir (4) eine Öffnung (5) aufweist, die ermöglicht, dass Gas bei Inhalation in das Reservoir (4) fließt.

3. Vorrichtung (1) gemäß Anspruch 1 oder 2, ferner umfassend einen Sammler (8) zum Sammeln der ausgeatmeten Luft, wobei der Sammler (8) Mittel zum Erhitzen von ausgeatmeter Luft umfasst.

4. Vorrichtung (1) gemäß einem der vorstehenden Ansprüche, ferner umfassend einen Sammler (8) zum Sammeln der ausgeatmeten Luft.

5. Vorrichtung (1) gemäß einem der vorstehenden Ansprüche, wobei die Luft zur Inhalation, die von dem Mittel (7) zur Bereitstellung von Luft zur Inhalation bereitgestellt wird, frei von Partikeln mit den gleichen Charakteristika wie die in dem zu inhalierenden Aerosol enthaltenen Partikel ist.

6. Vorrichtung (1) gemäß einem der vorstehenden Ansprüche, wobei es sich bei den Mitteln (6, 10) zur Bestimmung der Menge von Partikeln in inhaliertem Aerosol und der ausgeatmeten Luft um das gleiche Gerät handelt.

7. Vorrichtung (1) gemäß einem der vorstehenden Ansprüche, wobei das Mittel (3) zur Bereitstellung des zu inhalierenden Aerosols, das Partikel umfasst, dafür beschaffen ist, eine Konzentration von Partikeln von kleiner als 500.000 Partikel/cm³, vorzugsweise von 1.000 bis 50.000 Partikel/cm³, bereitzustellen.

8. Vorrichtung (1) gemäß einem der vorstehenden Ansprüche, ferner umfassend Mittel zum Trocknen des Aerosols auf unter 20 % relative Feuchtigkeit.

9. Vorrichtung (1) gemäß einem der vorstehenden Ansprüche, wobei das System von Ventilen (12) regelbar ist, um entweder Durchtreten von Luft aus dem Mittel (7) zur Bereitstellung von Luft zur Inhalation an das Subjekt zu erlauben, Durchtreten von Aerosol, das Partikel umfasst, aus dem Reservoir (4) zu dem Subjekt zu erlauben, Durchtreten von Aerosol, das Partikel umfasst, aus dem Reservoir (4) zu dem Mittel (6) zur Bestimmung der Menge von Partikeln in dem Aerosol zu erlauben, Durchtreten von ausgeatmeter Luft von dem Subjekt zu dem Mittel (10) zur Bestimmung der Menge von Partikeln in ausgeatmeter Luft zu erlauben oder Durchtreten von ausgeatmeter Luft von dem Subjekt an die Umgebung zu erlauben.

10. Vorrichtung (1) gemäß Anspruch 9, wobei das System von Ventilen (12) regelbar ist, um zu verhindern, dass inhaliertes Aerosol von dem Subjekt ausgeatmet wird.

## Revendications

1. Dispositif (1) pour une mesure de la fonction pulmonaire chez un sujet, le dispositif comprenant
des moyens (3) pour la génération d'aérosols comprenant des particules hydrophobes dans la plage de 20 à 200 nm, de préférence de 50 à 100 nm, devant être inhalés par le sujet, les moyens (3) pour la génération d'un aérosol comprenant un réservoir (4) fournissant une concentration/distribution uniformes de particules dans l'aérosol, les moyens (3) étant configurés pour faire passer les aérosols générés vers le réservoir (4),
des moyens (6, 10) pour déterminer la quantité de particules dans l'aérosol inhalé et l'air expiré, et
un système de valves pouvant être ouvertes vers le sujet, pouvant être ouvertes vers un moyen (7) pour fournir de l'air pour l'inhalation, pouvant être ouvertes vers le réservoir (4), et pouvant être ouvertes vers le moyen (6, 10) pour déterminer la quantité de particules dans un aérosol inhalé et l'air expiré,
dans lequel le système de valves permet de contrôler la partie d'air expiré qui est dirigée vers les moyens (10) pour déterminer la quantité de particules dans l'air expiré, dans lequel ladite partie est expirée après l'expiration d'un volume correspondant à un espace mort.

2. Dispositif (1) selon la revendication 1, le réservoir (4) possédant une ouverture (5) permettant qu'un gaz s'écoule dans le réservoir (4) lors de l'inhalation.

3. Dispositif (1) selon la revendication 1 ou 2, comprenant en outre un collecteur (8) pour collecter l'air expiré, ledit collecteur (8) comprenant des moyens (11) pour chauffer l'air expiré.

4. Dispositif (1) selon l'une quelconque des revendications précédentes, comprenant en outre un collecteur (8) pour collecter l'air expiré.

5. Dispositif (1) selon l'une quelconque des revendications précédentes, dans lequel ledit air pour l'inhalation fourni par ledit moyen (7) pour fournir de l'air pour l'inhalation est dépourvu de particules ayant les mêmes caractéristiques que les particules comprises dans l'aérosol à inhaler.

6. Dispositif (1) selon l'une quelconque des revendications précédentes, les moyens (6, 10) pour déterminer la quantité de particules dans l'aérosol inhalé et l'air expiré étant le même instrument.

7. Dispositif (1) selon l'une quelconque des revendications précédentes, dans lequel lesdits moyens (3) pour fournir l'aérosol comprenant des particules à inhaler sont conçus pour fournir une concentration de particules inférieure à 500 000 particules/cm³, de préférence 1 000 à 50 000 particules/cm³.

8. Dispositif (1) selon l'une quelconque des revendications précédentes, comprenant en outre des moyens pour sécher l'aérosol à une humidité relative inférieure à 20 %.

9. Dispositif (1) selon l'une quelconque des revendications précédentes, dans lequel le système de valves (12) est réglable soit pour permettre le passage de l'air depuis le moyen (7) pour fournir de l'air pour l'inhalation vers le sujet, soit pour permettre le passage de l'aérosol comprenant des particules depuis le réservoir (4) vers le sujet, soit pour permettre le passage de l'aérosol comprenant des particules depuis le réservoir (4) vers les moyens (6) pour déterminer la quantité de particules dans l'aérosol, soit pour permettre le passage de l'air expiré depuis le sujet vers les moyens (10) pour déterminer la quantité de particules dans l'air expiré, soit pour permettre le passage de l'air expiré depuis le sujet vers les environs.

10. Dispositif (1) selon la revendication 9, dans lequel le système de valves (12) est réglable pour empêcher que l'aérosol inhalé soit expiré par le sujet.
